(19) Europäisches Patentamt
European Patent Office
Office européen des brevets

(11) **EP 2 191 772 B1**

(12) **EUROPEAN PATENT SPECIFICATION**

(45) Date of publication and mention
of the grant of the patent:
**09.05.2018 Bulletin 2018/19**

(51) Int Cl.:
*A61B 5/0484* $^{(2006.01)}$   *A61B 5/0488* $^{(2006.01)}$

(21) Application number: **09176057.9**

(22) Date of filing: **16.11.2009**

(54) **Measurement of responsiveness of a subject**

Messung der Reaktionsfähigkeit eines Subjekts

Mesure de la réactivité d'un sujet

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **26.11.2008 US 323953**

(43) Date of publication of application:
**02.06.2010 Bulletin 2010/22**

(73) Proprietor: **General Electric Company**
**Schenectady, NY 12345 (US)**

(72) Inventors:
• **Viertio-Oja, Hanna**
**02660, Espoo (FI)**
• **Uutela, Kimmo**
**00100, Helsinki (FI)**
• **Virtanen, Juha**
**00660, Helsinki (FI)**

(74) Representative: **Illingworth-Law, William Illingworth**
**GPO Europe**
**GE International Inc.**
**The Ark**
**201 Talgarth Road**
**Hammersmith**
**London W6 8BJ (GB)**

(56) References cited:
**EP-A1- 1 618 840     EP-A2- 1 704 819**
**EP-A2- 1 785 889     WO-A1-2006/122349**

**Description**

**Background of the Invention**

This disclosure relates generally to the assessment of the responsiveness of a subject, especially the responsiveness of a subject with lowered level of consciousness.

One of the special applications of electroencephalography (EEG), which has received attention recently, is the use of a processed EEG signal for objective quantification of the amount and type of brain activity for the purpose of determining the level of consciousness of a patient. In its simplest form, the utilization of an EEG signal allows automatic detection of the alertness of an individual, i.e. if he or she is awake or asleep. This has become an issue of increased interest, both scientifically and commercially, in the context of measuring the level of unconsciousness induced by anesthesia during surgery.

Another important component of balanced anesthesia is analgesia, i.e. prevention of pain reactions of a patient by administration of pain medication. Adequate analgesia reduces surgical stress and there is firm evidence that it decreases postoperative morbidity. Awareness during surgery with insufficient analgesia may lead to a post-traumatic stress disorder. Low quality pre- and intra-operative analgesia makes it difficult to select the optimal pain management strategy later on. More specifically, it may cause exposure to unwanted side effects during the recovery from the surgery.

If the anesthesia is too light and involves insufficient level of unconsciousness, it may cause traumatic experiences both for the patient and for the anesthesia personnel. From an economical point of view, if the anesthesia is too deep, it may cause increased perioperative costs through extra use of drugs and time, and extend the time required for post-operative care.

Virtually every patient being cared for in an intensive care unit (ICU), for example, receives some form of sedation, i.e. administration of sedative drugs that aim to induce calmness, relaxation, and sleepiness. However, the control of the depth of the sedation administered to a patient is still problematic, and therefore oversedation and undersedation are both rather common occurrences in intensive care units. At present, monitoring the level of sedation is mainly handled by using subjective observations from the patient. Various observational scoring systems have been developed, in which the response of the patient to an intentionally generated stimulus is assessed subjectively. Such scoring systems include the Ramsay Scale, the Glasgow Coma Scale (GCS), and the Richmond Agitation Sedation Scale (RASS).

The level of (un)consciousness is not directly measurable. Therefore, drug delivery systems have to derive the level of (un)consciousness from a surrogate signal or from indirectly measured parameters. The most common and popular surrogate signal for this purpose is the EEG, from which several parameters may be determined. The basic reason for the insufficiency of a single parameter is the variety of drugs and the complexity of the drug effects on the EEG signal in human brains. However, during the past few years, some commercial validated devices for measuring the level of consciousness and/or awareness in clinical set-up during anesthesia or sedation have become available.

In addition to the EEG signal data, electromyographic (EMG) signal data obtained from facial muscles (fEMG) of the forehead is used for monitoring purposes during anesthesia. Recovering facial muscle activity is often the first indicator of the patient approaching consciousness. When this muscle activity is sensed by electrodes placed appropriately, it provides an early indication that the patient is emerging from anesthesia. Similarly, these electrodes can sense pain reactions when the anesthesia is not adequate due to inadequate analgesia. So, the EMG signals give an early warning of the arousal of the patient, and they may also be indicative of inadequate analgesia.

The lowered level of the consciousness of a patient is thus typically induced by one or more anesthetic and/or sedative drugs, but may also be caused by a neurological disorder. Recently, various automatic mechanisms have been suggested for assessing the responsiveness of a patient. In some systems, well-defined stimulus signals are supplied either automatically or manually to the patient and a measurement signal derived from the patient is monitored to determine how the characteristics of the measurement signal are changed in response to the stimulus signals. However, active stimulation provides only intermittent information and its use as a primary mechanism for assessing the responsiveness may be problematic if the patient needs rest and sleep. For example, getting enough sleep is a problem in the ICU, which limits the use of active stimulation for assessing the responsiveness.

In another approach for assessing the responsiveness, no active stimulation is used but responses in EEG and/or EMG signals to background stimulation are detected. Background stimulation here refers to the various types of unintentional stimulation signals to which the patient is inexorably exposed in the environment in which responsiveness is measured, such as clinical environment. Such unintentionally caused stimulation may originate from various sources, such as sound/noise sources, lights, caregiving procedures, and ventilation. Background stimulation may also originate internally if the patient is feeling pain or anxiety. The strength and frequency of the responses depend on the level of the background stimulation as well as on the state of the patient. As deepening sedation tends to suppress the naturally occurring arousals, a system measuring responses to background stimulation provides an automatic mechanism for assessing the depth of sedation relative to the stimulus level

Although the above mechanism that monitors responses to background stimulation provides valuable information for clinical decision-making, the information provided is not directly comparable to the information obtained from the scoring systems that are commonly regarded as the "golden standards" for the depth of sedation, such as the Ramsay scale or the RASS. This is at least partly due to the more or less sporadic nature of the background stimulation, which may obscure the real state of the patient. Therefore, it may sometimes be difficult to distinguish between the effect of medication and the effect of the environment on the state of the patient. For example, in certain situations the responsiveness determined based on background stimulation may show low values. This provides an indication for the clinician to check the level of sedative drug administration and to consider reducing it. However, low responsiveness may also be due to a preceding period of low background stimulation; if the patient is comfortable and has no pain, he/she may have fallen asleep. Thus, the nature of the preceding background stimulation may lead to a situation in which the effect of medication on the level of consciousness cannot be deduced directly from the measured responsiveness. Finally, reference is explicitly made to documents EP1618840 A1 and EP1785889 A2 disclosing prior art arrangements for measuring the responsiveness of a subject.

**Brief Description of the Invention**

The above-mentioned problems are addressed herein which will be comprehended from the following specification.

In an embodiment, there is provided an apparatus or arrangement for measuring the responsiveness of a subject as claimed in claim 1.

In a further embodiment, there is a computer program product for an apparatus monitoring the responsiveness of a subject as claimed in claim 4.

Various other features, objects, and advantages of the invention will be made apparent to those skilled in the art from the following detailed description and accompanying drawings.

**Brief Description of the Drawings**

FIG. 1 illustrates a method of measuring the responsiveness of a subject;
FIG. 2 illustrates the calculation of the indices of responsiveness in the embodiment of FIG. 1;
FIG. 3 illustrates the determination of high-frequency EEG/EMG power change in the embodiment of FIG. 2;
FIG. 4 illustrates an example of the index curves obtained in an embodiment shown in FIG. 2;
FIG. 5 is a two-dimensional plot illustrating the conclusions that may be drawn based on the concurrent magnitudes of the two indices of responsiveness;
FIG. 6 shows an embodiment of a system for measuring the responsiveness of a subject; and
FIG. 7 illustrates the operational units of the control unit of FIG. 6 for determining the two indices of responsiveness;

**Detailed Description of the Invention**

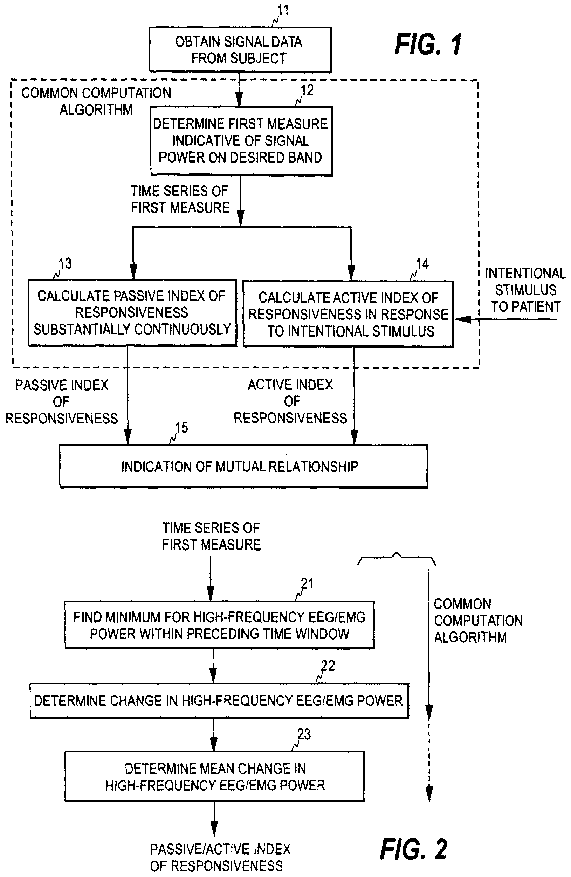

FIG. 1 illustrates a method for determining the responsiveness of a subject. Physiological signal data is first measured from the subject (step 11). Since the measured data is typically EEG and/or EMG signal data, it is assumed in the examples below that an EEG signal data is measured from the subject. The signal is first digitized and the sampled EEG signal may further be filtered to exclude high- and low-frequency artifacts (not shown in the figure). As is common in the art, the digitized signal samples are processed as sets of sequential signal samples representing finite time blocks or time windows, commonly termed "epochs".

Based on the signal data, the process then determines at step 12 a first measure descriptive of a desired property of the signal, such as the current high-frequency EEG power or the entropy of the signal, and stores the calculated value. The EEG/EMG power $P_{EEG/EMG}(t)$ is defined here as the squared value of the instantaneous signal amplitude $S_{EEG/EMG}(t)$ as $P_{EEG/EMG}(t) = (S_{EEG/EMG}(t))^2$, while the power of an epoch of a digitized signal may be defined as the average of such squared amplitudes within the epoch, for example. Instead of EEG entropy, another parameter that characterizes the amount of disorder or irregularity in the EEG signal data may be determined. Generally, the first measure determined may be directly or indirectly indicative of the level of consciousness of the subject. In the former case, high-frequency EEG power may be calculated, while in the latter case high-frequency EMG power may be calculated, for example. Henceforward, high-frequency EEG power is used as an example of the first measure.

The high-frequency EEG power may be derived by calculating the power of the signal data on a frequency band comprising high-frequency EEG components. The power may be derived from the power spectrum of the EEG signal by calculating, for example, the EEG power on a frequency band extending from 50 Hz to 150 Hz. The length of the time window within which the power is determined may correspond to 5 seconds (one epoch), for example. The Fast Fourier Transform, for example, is a computationally effective algorithm for this purpose. Alternatively, the high-frequency EEG power may be calculated straight from the time-domain signal, by utilizing appropriate filters.

A time series representing the first measure is thus obtained from step 12. Although high-frequency EMG power may be determined using the same parameters as in the determination of high-frequency EEG power, it is obvious that the values of the above parameters of high-frequency EEG/EMG power determination may change.

Based on the time series of the first measure, the process then determines two variables of responsiveness, which are in this context termed indices of responsiveness. A common computation algorithm may be used in the determination of the indices, as is shown in the figure. Here, the generation of the time series of the first measure may be regarded as part of the common algorithm, since both indices are determined based on the time series of the first measure. The purpose of the common algorithm is to make the two indices comparable to each other, so that conclusions can be made on state of the subject based on the values that the indices have substantially simultaneously. Although the common computation algorithm may define the whole determination process for each index or only part of each index determination process, any other mechanism that makes the two indices commensurable to each other may also be used. For example, two entirely separate computation algorithms may be used for the indices, if it can be mathematically proven that the outputs of the algorithms, i.e. the indices, are commensurable to each other. Next, the determination of the indices is discussed in more detail assuming that an algorithm is used which is partly or fully common for the determination of the two indices.

In a clinical environment a patient is continually exposed to the above-described background stimulation caused by various unintentional stimulation sources, which may be external (such as ventilation via an intubation tube, lights, noise, etc.) or internal (pain/discomfort). A first index of responsiveness is determined substantially continuously at step 13, the first index being caused by the background stimulation only. Further, a second index of responsiveness is determined intermittently at step 14 in response to a stimulation to which the subject is exposed intentionally. Due to the nature of the two types of stimulation, the first and second indices of responsiveness are in this context termed the passive and active index of responsiveness, respectively. The two indices are inherently in comparable proportion to each other and the system indicates the mutual relationship of the two indices to the user (step 15). Based on the simultaneous values of the two indices, the system or the clinician may make decisions on the state of the subject. The system may also determine a further variable indicative of the relative magnitudes of the indices, such as the ratio of the indices.

FIG. 2 illustrates an embodiment of the determination of the passive index in step 13. However, as discussed below, the steps of FIG. 2 may also apply to the determination of the active index. For the sake of clarity, the determination of the passive index is discussed first.

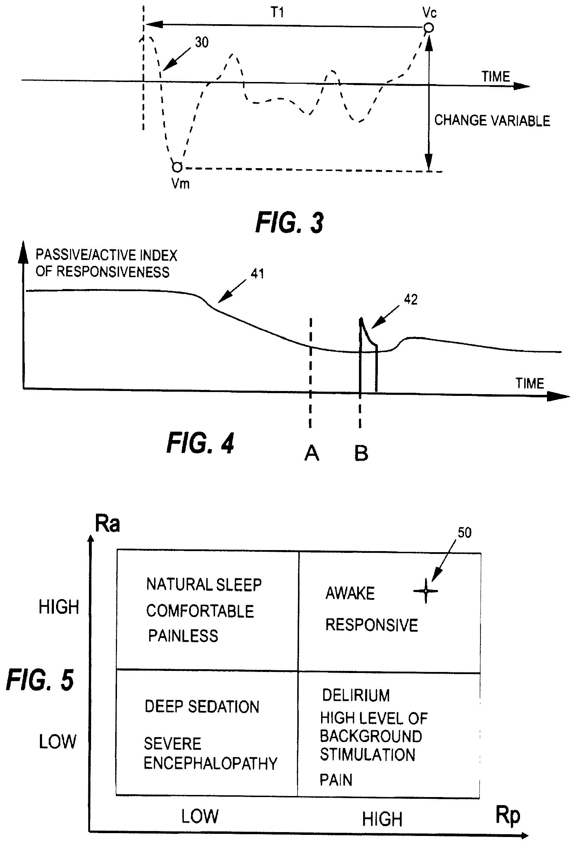

Step 13 first involves the determination of a change variable indicative of changes in the high-frequency EEG (or EMG) power, the changes being positive in this example. For the determination of the change variable, the process may first find the minimum high-frequency EEG power defined within a preceding time window of a predetermined length (step 21). The length of the time window from which the minimum is searched may be 1 minute, for example. The change variable is then determined by subtracting the minimum power value from the current power value (step 22). The determination of the change variable is illustrated in FIG. 3, in which curve 30 illustrates the set of distinct power values obtained during the preceding time window T1 from which the minimum is retrieved. If the current high-frequency EEG power is Vc, for example, the current value of the change variable corresponds to Vc-Vm.

With reference to FIG. 2 again, the values of the change variable obtained during another, longer time window T2 are then used to obtain the passive index indicative of the mean high-frequency EEG power changes with respect to time (step 23). The said time window T2 may be long with respect to the typical frequency of the unintentionally caused arousals of the patient. In this example, the passive index may be calculated over a time window of 30 minutes, for example. Thus, the window extends 30 minutes backwards from the current moment. Nevertheless, steps 12 and 21 to 23 may be carried out for each epoch.

Since the absolute value of the change variable may be high, the usability of the change variable may be enhanced by calculating the logarithm of the above-described difference. The passive index of responsiveness Rp may thus be determined in step 23 according to equation (1) as follows:

$$Rp(t) = \frac{1}{T2} \int_{t-T2}^{t} \log(\frac{\Delta P(u)}{P_0}) du, \quad (1),$$

*where*

$$\Delta P(u) = P_{EEG/EMG}(u) - \min_{(u-T1)\leq v\leq u} P_{EEG/EMG}(v)$$

where $P_{EEG/EMG}(ti)$ refers to high-frequency EEG or EMG power in a short time window ti computed over the desired frequency range, such as the above-mentioned range of 50 Hz to 150 Hz and $P_0$ is a fixed reference value for the power change. In the above example, the length of this time window corresponds to one epoch (5 seconds), while T2 may equal to 30 minutes and T1 to 1 minute, as discussed above. However, these parameter values may change.

The active index may be calculated in response to an intentional stimulus using the same raw function $\Delta P(u)$, but the active index is only computed for a restricted time following the time instant t=0 of the stimulus. When no stimuli are given, the active index is equal to zero. Furthermore, although the length of the time window for the active index may be fixed, it may also increase from zero to the value of T2', where T2' corresponds to the typical duration of a response. In this embodiment, all the above steps, except the averaging step 23, may thus be carried out by the common computation algorithm. If a fixed time window is used for the active index too, a common algorithm may be employed for the determination of both indices, only one parameter, i.e. the length of the fixed time window of the averaging step, is index-specific. While the time window for the averaging step for the passive index, T2, needs to be relative long, such as 30 minutes, to include a sufficient sample of background stimulation, the corresponding time window for the active index, T2', may be considerably shorter, such as a couple of minutes.

If the averaging step 23 is separate for the two indices, the time series of the first measure may thus be processed as shown in FIGs. 2 and 3, but in step 23 of FIG. 2 the active index may be computed based on the raw function $\Delta P(u)$ for an individual stimulus according to Eq. (2):

$$Ra(t) = \frac{1}{t}\int_0^t \log(\frac{\Delta P(u)}{P_0})du, \ (0 < t \leq T2'); \quad \text{and } Ra(t) = 0, \ (t < 0 \text{ or } t > T2'), \ (2)$$

where $\Delta P(u) = P_{EEG/EMG}(u) - \min_{(u-T1)\leq v\leq u} P_{EEG/EMG}(v)$ and

where t=0 corresponds to the time instant at which the intentional stimulus is given and $P_0$ is a fixed reference value for the power change.

Since the function $\Delta P(u)$, which is determined in steps 12, 21, and 22, remains the same for each index determined even if the averaging step 23 is different, the indices are inherently in comparable proportion to each other. Therefore, a significant response to an active stimulus results in a value of Ra that is significantly greater than the (substantially simultaneous) value of Rp.

Due to the above subtraction included in the raw function $\Delta P(u)$ in this embodiment, the indices do not depend on the absolute level of the high-frequency EEG. The averaging step may also involve calculation of a weighted average, for example so that more recent values obtain a higher weight than less recent (older) values.

Various other implementations for the indices Ra and Rp are also possible. For example, the function $\Delta P(u)$ defined in equations (1) and (2) may alternatively be defined as

$$\Delta P(u) = P_{EEG/EMG}^{rms}(u) - P_{EEG/EMG}^{rms}(u-v) \ \text{ when } P_{EEG/EMG}^{rms}(u) - P_{EEG/EMG}^{rms}(u-v) > 0;$$

and

$$\Delta P(u) = 0 \ \text{when} \ P_{EEG/EMG}^{rms}(u) - P_{EEG/EMG}^{rms}(u-v) \leq 0$$

where $P_{EEG/EMG}^{rms}(u) = \sqrt{P_{EEG/EMG}(u)}$ and v is the time lag used for the derivation of a change in the function $P_{EEG/EMG}^{rms}(u)$, for example v = 20 seconds. The integral expression $\int \log(\frac{\Delta P(u)}{P_0})du$ may be replaced, for example, by the expression $\int \log((\frac{\Delta P(u)}{P_0})^2 + 1)du$. Thus, in this embodiment the change variable indicative of (positive) changes in the first measure is not determined by searching a minimum from a preceding time window, but based on a fixed time lag v. In other words, this embodiment does not include step 21 of FIG. 2, but only step 22, which now includes defining $\Delta P(u)$ as shown above, and the averaging step 23.

FIG. 4 illustrates an example of the index values obtained through Eqs. (1) and (2). Curve 41 shows the passive index computed according to Eq. (1), while curve 42 shows the active index computed according to Eq. (2) in response to a stimulus given at time instant B. In this example, a light stimulus given at time instant A does not result in an observable response. The determination of the active index may be triggered manually by a clinician at desired time instant(s) and/or automatically by the measurement device at regular intervals of time, for example, such as once in an hour. The determination of the passive index does not have to be interrupted when the determination of the active index is started, i.e. when an intentional stimulus is given. This is also the case in the example of FIG. 4, where the stimulus given at time instant B is reflected in curve 21 too. The types of the active stimuli may vary but are typically chosen according to the stimulation mode. In case of automatic stimulation, the stimuli may be suitable for automatic generation, such as "open your eyes" command or another audio stimulus supplied through headphones. In case of manual stimulation, stimuli according to the RASS may be used, for example, which the clinician may give simultaneously as (s)he indicates the time instant of the stimulus to the measurement device by pushing a button, for example.

The calculation of the common raw function may change depending, for example, on the physiological signal and the frequency band used. However, the raw signal is typically based on the amplitude of the physiological signal measured from the subject.

The information given to the clinician may vary. In a simple embodiment, the measurement device displays the values of the two indices. Since the two indices are inherently in comparable proportion to each other, the clinician may assess, based on the relative magnitudes of the indices, the state of the subject and/or the effect of the medication on the subject. If, for example, the passive index is low and the active index is at the same time rather high, it is likely that the patient is painless and may be in natural sleep.

In another embodiment, the system may indicate the subject's location on a two dimensional plot, such as a quadrant of a coordinate system, in which one axis represents the passive index and the other axis the active index. FIG. 5 illustrates such a plot divided into four zones based on the magnitudes of the indices. Each zone in the figure indicates the conclusions that may be made if the subject is located in that zone. In one embodiment, the measured position of the subject in the coordinate system may be displayed as is shown by a star 50 in the figure. The conclusions corresponding to each zone may or may not be shown to a user. Alternatively, a separate message field may be used to indicate what the user should consider based on the measured position of the subject in the two dimensional plot.

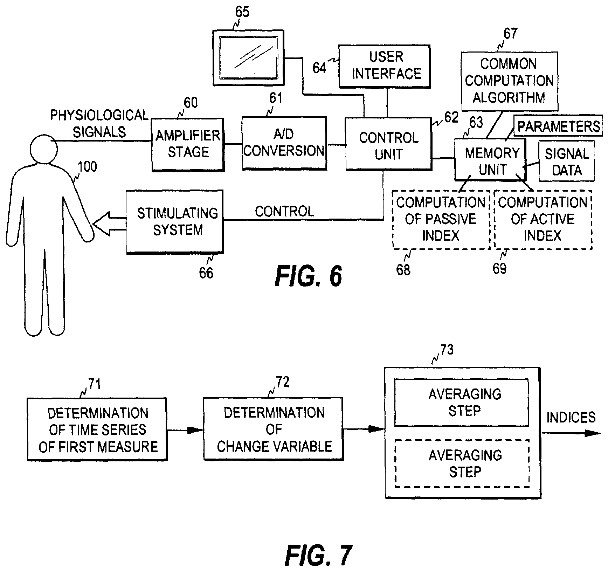

FIG. 6 illustrates one embodiment of the system or apparatus for measuring the responsiveness of a subject. The physiological signal(s) obtained from one or more sensors attached to a subject 100 are supplied to an amplifier stage 60, which amplifies the signal(s) before they are sampled and converted into digitized format in an A/D converter 61. The digitized signals are supplied to a control unit 62 which may comprise one or more processors. As noted above, the signal data measured from the subject is typically EEG signal data, which may be measured through electrodes applied to the forehead of the subject. The electrodes also receive high-frequency EEG signals and the received signal may include EMG signal components. Requirements for artifact detection procedures to be carried out in control unit 62 depend on the choice of the frequency range used.

The control unit is provided with a memory or database 63 holding the digitized signal data obtained from the sensor(s). The memory or database may also store a computation algorithm 67 that is common for the determination of both indices of responsiveness. Moreover, if the active index is determined according to Eq. (2), for example, the memory or database may also store separate algorithms 68 and 69 for the averaging steps 23, i.e. algorithms for determining the indices according to Equations (1) and (2) based on the common raw function $\Delta P(u)$. The memory or database also may store the parameters of the algorithms, such as the values of the window lengths and frequency limits.

The system may further be provided with a stimulating system 66 for giving the stimulation signals needed for the measurement of the active index of responsiveness. The stimulating system may be controlled by the control unit or by the user through the user interface 64 (and the control unit). The stimulating system may generate any type of predefined stimuli suitable for determining the active index, such as audio, mechanic, chemical, magnetic, or electric stimuli. Furthermore, one or more stimulus signals may be given for one measurement period of the active index. For example, the stimulus may be an electric TOF (train-of-four) stimulus applied to a peripheral motor nerve. The control unit may automatically trigger the measurement of the active index at regular intervals, such as every hour or half an hour.

As is illustrated in FIG. 7, the control unit, which is equipped with the above computation algorithms, may be seen as an entity of three consecutive operational modules or units for determining the two indices: a first computation unit 71 configured to generate the time series of the first measure based on the physiological signal data acquired from the subject, a second computation unit 72 configured to determine the time series of the change variable, and a third computation unit 73 for carrying out the averaging step 23 to obtain the final indices. The values of the raw function $\Delta P(u)$ of Eq. (1) and (2) are thus computed in unit 72, while the averaging step of the said equations is carried out in unit 73. As discussed above, the averaging step may be common or specific to each index. Thus, although the system or apparatus for measuring the responsiveness comprises two determination units in logical sense, one for determining the passive index and another for determining the active index, the two units may share common hardware and a common calculation algorithm portion, which makes them separate only in logical sense.

The resulting index values obtained from unit 73 may be displayed on the screen of a monitor 65. The responsiveness curves may also be displayed graphically as is shown in FIG. 4. Moreover, information concerning the conclusions that the system makes based on the magnitudes of the simultaneous index values may be displayed to the user. The control unit may also determine a further variable to be displayed to the user in addition to or instead of the index values, such as the difference or ratio of the two index values. The said further variable may also be averaged over an appropriate time window.

Although one computer unit or processor may perform the above steps, the processing of the physiological data may also be distributed among different units/processors (servers) within a network, such as a hospital LAN (local area network). The apparatus of the invention may thus also be implemented as a distributed system.

As discussed above, the system may act as a decision-support tool for the clinician. For example, the clinician may control the operation of a drug delivery system based on the concurrent magnitudes of the two indices. The system may also control the drug delivery system automatically according to the index values and an internal set of decision rules.

A conventional patient monitor that acquires EEG (or EMG) signal data from a subject may also be upgraded to determine the two indices of responsiveness. Such an upgrade may be implemented by delivering to the patient monitor a plug-in software module that enables the device to calculate the two indices based on the data acquired by the device. The software module may be delivered, for example, on a data carrier, such as a CD or a memory card, or through a telecommunications network. Logically, the software module includes two units: a first program code portion for determining the passive index indicative of responsiveness of the subject to unintentional background stimulation affecting the subject and a second program code portion configured to define the active index indicative of responsiveness of the subject. However, the two program portions are not necessarily separate, since they are configured to make the two indices commensurable to each other, and may thus employ a common program code portion for this purpose. As discussed above, the portion of the software module that is common to the index determination processes, i.e. to the said two program code portions, may change depending on the implementation of the averaging process, for example. Furthermore, the two program portions may be separate, if it can be mathematically proven that the outputs of the corresponding algorithms, i.e. the indices, are commensurable to each other.

The software module may also comprise a new software version for the device, which replaces the existing software of the device. Thus, the software module may comprise, in addition to the algorithms described above, the algorithms of a patient monitor, such as the algorithms for determining the level of consciousness of the subject.

A responsiveness monitor may also be implemented as a separate module connectable to a conventional patient monitor, such as a monitor intended for measuring the level of consciousness. Such a responsiveness monitor may comprise a data processing unit that receives EEG or EMG data or the time series of the first measure from the conventional patient monitor and derives the two indices based on the received data. Such a responsiveness monitor may comprise a display of its own for displaying the computed indices to the user, and it is further provided with a stimulating unit and/or a triggering device for triggering the measurement of the active index.

This written description uses examples to disclose the invention, including the best mode, and also to enable any person skilled in the art to make and use the invention. The patentable scope of the invention is defined by the claims.

## Claims

1. An arrangement for measuring the responsiveness of a subject (100), the arrangement comprising:

   - a first determination unit (62; 71-73) configured to determine, based on physiological signal data obtained from a subject (100), a first index indicative of responsiveness of the subject to unintentional background stimulation affecting the subject; and
   - a second determination unit (62; 71-73) configured to define, based on the physiological signal data, a second index indicative of responsiveness of the subject (100) to intentional stimulation,

   wherein the first determination unit and the second determination unit are configured to make the first index and the second index commensurable to each other by recording a time series of a first measure indicative of a desired property of the physiological signal data and generating a time series of a change variable indicative of short-time changes in the first measure, both indexes being determined based on said change variable;
   **characterized in that** the first index is determined substantially continuously, the first index at a given instant being obtained based on the values of the change variable obtained during a first time window extending backwards from said given instant, the first time window being sufficiently long to include a sufficient sample of background stimulation, and
the second index is determined intermittently in response to an intentional stimulus, said second index being obtained based on the values of the change variable obtained during a second time window following to the instant of application of an intentional stimulus, the second time window being considerably shorter than the first time window.

2. The arrangement according to claim 1, wherein

- the physiological signal data includes at least one of EEG signal data and EMG signal data; and

wherein the desired property represents one of power and entropy of the physiological signal data on a predetermined frequency band.

3. The arrangement according to claim 1, further comprising a display unit (65) configured

- to determine a location corresponding to current values of the first index and the second index on a two dimensional plane defined by the first index and the second index and to present the location to a user; or

to generate a message to a user based on concurrent values of the first index and the second index.

4. A computer program product for an apparatus monitoring the responsiveness of a subject (100), the computer program product comprising:

- a first program code portion (67, 68) configured to determine, based on physiological signal data obtained from a subject (100), a first index indicative of responsiveness of the subject to unintentional background stimulation affecting the subject; and
- a second program code portion (67, 69) configured to define, based on the physiological signal data, a second index indicative of responsiveness of the subject (100) to intentional stimulation,

wherein the first program code portion and the second program code portion are configured to make the first index and the second index commensurable to each other by recording a time series of a first measure indicative of a desired property of the physiological signal data and generating a time series of a change variable indicative of short-time changes in the first measure, both indexes being determined based on said change variable; the first program code portion being configured such that the first index is determined substantially continuously, the first index at a given instant being obtained based on the values of the change variable obtained during a first time window extending backwards from said given instant, the first time window being sufficiently long to include a sufficient sample of background stimulation, and the second program code portion being configured such that the second index is determined intermittently in response to an intentional stimulus, said second index being obtained based on the values of the change variable obtained during a second time window following to the instant of application of an intentional stimulus, the second time window being considerably shorter than the first time window.

**Patentansprüche**

1. Anordnung zum Messen der Reaktionsfähigkeit eines Subjekts (100), wobei die Anordnung umfasst:

- eine erste Bestimmungseinheit (62; 71-73), die konfiguriert ist, um basierend auf physiologischen Signaldaten, die von einem Subjekt (100) erhalten werden, einen ersten Index zu bestimmen, der die Reaktionsfähigkeit des Subjekts auf unbeabsichtigte Hintergrundstimulation angibt, die das Subjekt beeinflusst; und
- eine zweite Bestimmungseinheit (62; 71-73), die konfiguriert ist, um basierend auf den physiologischen Signaldaten einen zweiten Index zu definieren, der die Reaktionsfähigkeit des Subjekts (100) auf absichtliche Stimulation angibt,

wobei die erste Bestimmungseinheit und die zweite Bestimmungseinheit konfiguriert sind, um den ersten Index und den zweiten Index miteinander vergleichbar zu machen, indem eine Zeitserie einer ersten Messung, die eine gewünschte Eigenschaft der physiologischen Signaldaten angibt, aufgezeichnet wird, und eine Zeitserie einer Veränderungsvariablen erzeugt wird, die Kurzzeitveränderungen in der ersten Messung angibt, wobei beide Indices basierend auf der Veränderungsvariablen bestimmt werden;

**dadurch gekennzeichnet, dass** der erste Index im Wesentlichen kontinuierlich bestimmt wird, wobei der erste Index zu einem gegebenen Zeitpunkt basierend auf den Werten der Veränderungsvariablen erhalten wird, die während eines ersten Zeitfensters, das sich von dem gegebenen Zeitpunkt rückwärts erstreckt, erhalten werden, wobei das erste Zeitfenster ausreichend lang ist, um eine ausreichende Probe der Hintergrundstimulation einzuschließen, und

dadurch, dass der zweite Index unterbrochen als Reaktion auf einen beabsichtigten Stimulus bestimmt wird, wobei der zweite Index basierend auf den Werten der Veränderungsvariablen erhalten wird, die während eines zweiten Zeitfensters erhalten werden, das dem Zeitpunkt der Anwendung eines beabsichtigten Stimulus folgt, wobei das zweite Zeitfenster bedeutend kürzer als das erste Zeitfenster ist.

2. Anordnung nach Anspruch 1, wobei

    - die physiologischen Signaldaten mindestens eines von EEG-Signaldaten und EMG-Signaldaten einschließen; und

    wobei die gewünschte Eigenschaft eines von Leistung und Entropie der physiologischen Signaldaten in einem vorbestimmten Frequenzband darstellt.

3. Anordnung nach Anspruch 1, ferner umfassend eine Anzeigeeinheit (65), die konfiguriert ist, um

    - einen Ort zu bestimmen, der den derzeitigen Werten des ersten Index und des zweiten Index auf einer zweidimensionalen Ebene entspricht, die durch den ersten Index und den zweiten Index definiert wird, und den Ort einem Benutzer vorzulegen; oder

    basierend auf zeitgleichen Werten des ersten Index und des zweiten Index eine Nachricht an einen Benutzer zu erzeugen.

4. Computerprogrammprodukt für eine Vorrichtung, welche die Reaktionsfähigkeit eines Subjekts (100) überwacht, wobei das Computerprogrammprodukt umfasst:

    - einen ersten Programmcodeabschnitt (67, 68), der konfiguriert ist, um basierend auf physiologischen Signaldaten, die von einem Subjekt (100) erhalten werden, einen ersten Index zu bestimmen, der die Reaktionsfähigkeit des Subjekts auf unbeabsichtigte Hintergrundstimulation angibt, die das Subjekt beeinflusst; und
    - einen zweiten Programmcodeabschnitt (67, 69), der konfiguriert ist, um basierend auf den physiologischen Signaldaten einen zweiten Index zu definieren, der die Reaktionsfähigkeit des Subjekts (100) auf absichtliche Stimulation angibt,

    wobei der erste Programmcodeabschnitt und der zweite Programmcodeabschnitt konfiguriert sind, um den ersten Index und den zweiten Index miteinander vergleichbar zu machen, indem eine Zeitserie einer ersten Messung, die eine gewünschte Eigenschaft der physiologischen Signaldaten angibt, aufgezeichnet wird, und eine Zeitserie einer Veränderungsvariablen erzeugt wird, die Kurzzeitveränderungen in der ersten Messung angibt, wobei beide Indices basierend auf der Veränderungsvariablen bestimmt werden;
    wobei der erste Programmcodeabschnitt derart konfiguriert ist, dass der erste Index im Wesentlichen kontinuierlich bestimmt wird, wobei der erste Index zu einem gegebenen Zeitpunkt basierend auf den Werten der Veränderungsvariablen erhalten wird, die während eines ersten Zeitfensters erhalten werden, das sich von dem gegebenen Zeitpunkt rückwärts erstreckt, wobei das erste Zeitfenster ausreichend lang ist, um eine ausreichende Probe der Hintergrundstimulation einzuschließen, und
    der zweite Programmcodeabschnitt derart konfiguriert ist, dass der zweite Index unterbrochen als Reaktion auf einen beabsichtigten Stimulus bestimmt wird, wobei der zweite Index basierend auf den Werten der Veränderungsvariablen erhalten wird, die während eines zweiten Zeitfensters erhalten werden, das dem Zeitpunkt der Anwendung eines beabsichtigten Stimulus folgt, wobei das zweite Zeitfenster bedeutend kürzer als das erste Zeitfenster ist.

**Revendications**

1. Agencement de mesure de la réactivité d'un sujet (100), l'agencement comprenant :

    - une première unité de détermination (62 ; 71-73) configurée pour déterminer, sur la base de données de signaux physiologiques obtenues auprès d'un sujet (100), un premier indice indicatif de la réactivité du sujet à une stimulation de fond involontaire affectant le sujet ; et
- une seconde unité de détermination (62 ; 71-73) configurée pour définir, sur la base des données de signaux physiologiques, un second indice indicatif de la réactivité du sujet (100) à une stimulation volontaire,

dans lequel la première unité de détermination et la seconde unité de détermination sont configurées pour rendre le premier indice et le second indice mutuellement commensurables en enregistrant une série temporelle d'une première mesure indicative d'une propriété souhaitée des données de signaux physiologiques et en générant une série temporelle d'une variable de changement indicative de changements à court terme de la première mesure, les deux indices étant déterminés sur la base de ladite variable de changement ;
**caractérisé en ce que** le premier indice est déterminé sensiblement en continu, le premier indice à un moment donné étant obtenu sur la base des valeurs de la variable de changement obtenues au cours d'une première fenêtre temporelle s'étendant en arrière depuis ledit moment donné, la première fenêtre temporelle étant suffisamment longue pour inclure un échantillon suffisant de stimulation de fond, et
le second indice est déterminé de manière intermittente en réponse à une stimulation volontaire, ledit second indice étant obtenu sur la base des valeurs de la variable de changement obtenues au cours d'une seconde fenêtre temporelle suivant le moment d'application d'une stimulation volontaire, la seconde fenêtre temporelle étant considérablement plus courte que la première fenêtre temporelle.

2.  Agencement selon la revendication 1, dans lequel :

    - les données de signaux physiologiques comprennent au moins l'une de données de signaux EEG et de données de signaux EMG ; et

    dans lequel la propriété souhaitée représente l'une de la puissance et de l'entropie des données de signaux physiologiques sur une bande de fréquences prédéterminée.

3.  Agencement selon la revendication 1, comprenant en outre une unité d'affichage (65) configurée pour :

    - déterminer un emplacement correspondant à des valeurs courantes du premier indice et du second indice sur un plan bidimensionnel défini par le premier indice et le second indice et présenter l'emplacement à un utilisateur ; ou
    - générer un message à un utilisateur sur la base de valeurs concurrentes du premier indice et du second indice.

4.  Produit de programme d'ordinateur pour un appareil surveillant la réactivité d'un sujet (100), le produit de programme d'ordinateur comprenant :

    - une première partie de code de programme (67, 68) configurée pour déterminer sur la base de données de signaux physiologiques obtenues auprès d'un sujet (100) un premier indice indicatif de la réactivité du sujet à une stimulation de fond involontaire affectant le sujet ; et
    - une seconde partie de code de programme (67, 69) configurée pour définir sur la base des données de signaux physiologiques un second indice indicatif de la réactivité du sujet (100) à une stimulation volontaire,

    dans lequel la première partie de code de programme et la seconde partie de code de programme sont configurées pour rendre le premier indice et le second indice mutuellement commensurables en enregistrant une série temporelle d'une première mesure indicative d'une propriété souhaitée des données de signaux physiologiques et en générant une série temporelle d'une variable de changement indicative de changements à court terme de la première mesure, les deux indices étant déterminés sur la base de ladite variable de changement ;
    la première partie de code de programme étant configurée de sorte que le premier indice soit déterminé de manière sensiblement continue, le premier indice à un moment donné étant obtenu sur la base des valeurs de la variable de changement obtenues au cours d'une première fenêtre temporelle s'étendant en arrière depuis ledit moment donné, la première fenêtre temporelle étant suffisamment longue pour inclure un échantillon suffisant de stimulation de fond, et
    la seconde partie de code de programme étant configurée de sorte que le second indice soit déterminé de manière intermittente en réponse à une stimulation volontaire, ledit second indice étant obtenu sur la base des valeurs de la variable de changement obtenues au cours d'une seconde fenêtre temporelle après le moment d'application d'une stimulation volontaire, la seconde fenêtre temporelle étant considérablement plus courte que la première fenêtre temporelle.

*FIG. 1*

OBTAIN SIGNAL DATA
FROM SUBJECT

11

COMMON COMPUTATION
ALGORITHM

DETERMINE FIRST MEASURE
INDICATIVE OF SIGNAL
POWER ON DESIRED BAND

12

TIME SERIES OF
FIRST MEASURE

CALCULATE PASSIVE INDEX OF
RESPONSIVENESS
SUBSTANTIALLY CONTINUOUSLY

13

CALCULATE ACTIVE INDEX OF
RESPONSIVENESS IN RESPONSE
TO INTENTIONAL STIMULUS

14

INTENTIONAL
STIMULUS
TO PATIENT

PASSIVE INDEX
OF
RESPONSIVENESS

ACTIVE INDEX
OF
RESPONSIVENESS

INDICATION OF MUTUAL RELATIONSHIP

15

TIME SERIES OF
FIRST MEASURE

COMMON
COMPUTATION
ALGORITHM

FIND MINIMUM FOR HIGH-FREQUENCY EEG/EMG
POWER WITHIN PRECEDING TIME WINDOW

21

DETERMINE CHANGE IN HIGH-FREQUENCY EEG/EMG POWER

22

DETERMINE MEAN CHANGE IN
HIGH-FREQUENCY EEG/EMG POWER

23

PASSIVE/ACTIVE INDEX
OF RESPONSIVENESS

*FIG. 2*

**FIG. 3**

**FIG. 4**

**FIG. 5**

| | | | |
|---|---|---|---|
| **HIGH** | NATURAL SLEEP COMFORTABLE PAINLESS | AWAKE RESPONSIVE | |
| **LOW** | DEEP SEDATION SEVERE ENCEPHALOPATHY | DELIRIUM HIGH LEVEL OF BACKGROUND STIMULATION PAIN | |
| | LOW | HIGH | **Rp** |

**FIG. 6**

**FIG. 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1618840 A1
- EP 1785889 A2